# EUROPEAN PATENT APPLICATION

(11) **EP 3 970 762 A1**
(43) Date of publication of application: **23.03.2022**
(21) Application number: 19928400.1
(22) Date of filing: 15.05.2019
(51) Int. Cl.: A61L 27/50, A61L 27/30, A61L 27/14, A61F 2/06, A61F 2/00, C23C 14/20, C23C 14/34, C23C 14/35

(54) **METHOD FOR MANUFACTURING EPTFE ARTIFICIAL BLOOD VESSELS HAVING IMPROVED HEMOCOMPATIBILITY VIA SELECTIVE PLASMA ETCHING**

(71) Applicant: Seoul National University R&DB Foundation, Seoul 08826 (KR); Genoss Co., Ltd., Gyeonggi-do 16229 (KR)
(72) Inventor: KIM, Hyoun-Ee, Seoul 06663 (KR); PARK, Cheonil, Seoul 08826 (KR); CHUNG, Sung Min, Suwon-si, Gyeonggi-do 16229 (KR)
(74) Representative: f & e patent
(86) International application number: PCT/KR2019/005834
(87) International publication number: WO 2020/230925

(57) **Abstract**

The present invention relates to a method of manufacturing an artificial vascular graft, which comprises implanting a bioactive metal into an expanded polytetrafluoroethylene (ePTFE) surface without an interface by performing plasma etching using a bioactive metal target, and an artificial vascular graft with improved blood compatibility, which is manufactured by way of the method.

## Description

### [Technical Field]

The present invention relates to a method of manufacturing an artificial vascular graft, which comprises implanting a bioactive metal into an expanded polytetrafluoroethylene (ePTFE) surface without an interface by performing plasma etching using a bioactive metal target, and an artificial vascular graft with improved blood compatibility, which is manufactured by way of the method.

### [Background Art]

When cholesterol or fat accumulates on the inner walls of blood vessels such as arteries or veins in the body and the blood vessels become partially blocked and harden, smooth blood circulation is hindered, causing various vascular diseases such as angina pectoris, myocardial infarction, and coronary artery disease. Recently, there has been a sharp increase in the occurrence of such vascular diseases, especially with increases in diabetes or obesity due to unbalanced diets and sedentary lifestyles in developed countries. Such vascular diseases have been reported as often being deadly, as once blood vessels have been blocked and their blood flow disturbed, the fatality rate is quite high. While non-surgical treatment methods for vascular diseases do exist, such as a method of inhibiting additional stenosis by administration of antianginal drugs, these drug treatments do not fundamentally resolve the issue of the stenotic site, and the pain caused by angina pectoris still persists despite continued administration of such drugs. Accordingly, surgical treatment methods are emerging as methods of treating vascular diseases, such as vascular grafting and vascular bypass, where surgery is performed to remove hardened blood vessels and connect artificial vascular grafts at the site of removal.

In such treatment methods via surgical operation, initially, an artery or vein of a donor was collected and transplanted, but this suffered from a low rate of success due to the occurrence of rejection or hardening. In contrast, autografting, where the patient's own healthy blood vessels are collected to replace the damaged portion, enjoys low immune response after transplantation and has the highest success rate, but this method has disadvantages in that the number of blood vessels that can be secured at a time is limited, and further surgery is required for blood vessel collection, increasing the burden to the patient. Therefore, in order to solve this problem, surgery has recently been performed to replace existing damaged blood vessels by using artificial vascular grafts made of synthetic polymers. Synthetic polymers that may be chosen for application to such artificial vascular grafts must first of all be harmless to the human body, and should also be materials that exhibit high biocompatibility. Furthermore, they must not be rejected by the immune system when transplanted into the body, and need to be able to be maintained in the body for a long period of time. In addition, in order to prevent potential post-transplantation stenosis and hardening of blood vessels, such synthetic polymers should not cause the precipitation of proteins or lipids, nor clotting and inflammatory response.

Having been selected in consideration of the above requirements, the material that is currently commercialized and used as an artificial vascular graft material is the fluoropolymer expanded polytetrafluoroethylene (ePTFE). This is a material which has been modified to have a micron-scale microporous structure by addition of an expanding step in the existing process of manufacturing polytetrafluoroethylene. Being a fluoropolymer, ePTFE is remarkably stable in the body because of its high surface hydrophobicity, and since it has no reactivity with other substances, the adhesion of thrombus to the inner wall of blood vessels can be inhibited. At the same time, the micropore structures of ePTFE provide passages between the inner and outer walls of blood vessels and promote the absorption of nutrients in the blood, and ePTFE is thus known as a material suitable for artificial vascular grafts. Further, in actual clinical results, high success rates have been reported in the transplantation of large-diameter artificial vascular grafts, with a diameter of 6 mm or more, and this is therefore being applied as a relatively stable surgical method.

However, in the case of small-diameter artificial vascular grafts with a diameter of 6 mm or less, stenosis still occurs at a high rate because of the low blood flow rate when an artificial vascular graft composed only of ePTFE material is transplanted. Therefore, research is underway to further improve blood compatibility by modification of the inner walls of ePTFE artificial vascular grafts. As specific examples, Korean Patent No. 10-1483846 discloses a method of enhancing blood compatibility by treating the inner walls of a polymer tube with a microplasma and imparting reactivity to the inner surface, while U.S. Patent No. 6,306,165 discloses a method of inhibiting clotting by coating the inner walls of an ePTFE artificial vascular graft with heparin, a representative antithrombotic protein.

However, even though it can improve biocompatibility, the conventional method of surface modification using a plasma has limitations in that the surface stability may be diminished due to the high reactivity of radicals generated after plasma treatment, and aging treatment is required in order to maintain the surface stability. Meanwhile, the method of coating with natural polymers or drugs such as anticoagulants suffers from disadvantages such as difficulty in maintaining the drug effect for a long period of time, since the hydrophobicity of the surface makes the binding force with these drugs weak, causing these drugs to be released in a short period of time with the flow of blood. In the case of the anticoagulant heparin, its excessive release leads to a decrease in platelets, and there is a risk of developing heparin-induced thrombocytopenia (HIT), which may cause serious secondary diseases depending on the age or health of the patient.

### [Disclosure]

### [Technical Problem]

The present inventors have made intensive research efforts to discover a surface modification method to improve the blood compatibility of the surface so that stenosis is not caused when an artificial vascular graft having a small diameter of 6 mm or less is used as well in the manufacture of an artificial vascular graft based on ePTFE, and as a result, they confirmed that it is possible to provide a surface on which the adhesion and/or proliferation of vascular endothelial cells is significantly increased and adhesion and/or proliferation of activated platelets is inhibited by implanting a bioactive metal into the ePTFE surface without an interface by way of selective plasma etching, thereby completing the present invention.

### [Technical Solution]

An object of the present invention is to provide a method of manufacturing an artificial vascular graft with a metallized surface comprising an expanded polytetrafluoroethylene (ePTFE) specimen and a bioactive metal implanted into a surface of the ePTFE specimen without an interface, which comprises applying a negative voltage to a bioactive metal target in the presence of an inert gas and at the same time applying a negative voltage for bias to a fixing plate on which an ePTFE specimen is positioned, thereby accelerating a bioactive metal cation by way of a potential difference formed between the bioactive metal target and the ePTFE specimen as a step of implanting a bioactive metal into an ePTFE surface without an interface by performing plasma etching using a bioactive metal target under a predetermined reaction condition.

Another object of the present invention is to provide an artificial vascular graft with a metallized surface, which comprises an ePTFE substrate; and a bioactive metal implanted into a surface of the ePTFE substrate without an interface.

### [Advantageous Effects]

The artificial vascular graft with a metallized surface, which comprises a bioactive metal implanted into a surface of an ePTFE specimen without an interface by way of selective plasma etching of the present invention exhibits improved blood compatibility that does not cause stenosis when used as an artificial vascular graft having a small diameter of 6 mm or less since the artificial vascular graft provides a surface on which the adhesion and/or proliferation of vascular endothelial cells is significantly increased and adhesion and/or proliferation of activated platelets is inhibited.

### [Brief Description of Drawings]

FIG. 1 is a diagram illustrating scanning electron microscope (SEM) images of an ePTFE surface before and after being treated by way of selective plasma etching;
FIG. 2 is a diagram illustrating the analytical results by X-ray photoelectron spectroscopy (XPS) of the chemical bonding states of atoms on an ePTFE surface before and after being treated by way of selective plasma etching;
FIG. 3 is a diagram illustrating the water contact angle on an ePTFE surface before and after being treated by way of selective plasma etching;
FIG. 4 is a diagram illustrating images of adhered vascular endothelial cells observed under a confocal microscope when adhered vascular endothelial cells are cultured for 1 day and 7 days on an ePTFE surface before and after being treated by way of selective plasma etching;
FIG. 5 is a diagram illustrating images of adhered platelets observed under SEM at various magnifications when platelets are cultured for 1 day on an ePTFE surface before and after being treated by way of selective plasma etching; and
FIG. 6 is a diagram illustrating the density of platelets adhered to an ePTFE surface before and after being treated by way of selective plasma etching when platelets are cultured for 1 day on the ePTFE surface.

### [Best Mode for Implementation of the Invention]

A first aspect of the present invention provides a method of manufacturing an artificial vascular graft with a metallized surface comprising an expanded polytetrafluoroethylene (ePTFE) specimen and a bioactive metal implanted into a surface of the ePTFE specimen without an interface, the method comprising applying a negative voltage to a bioactive metal target in the presence of an inert gas and at the same time applying a negative voltage for bias to a fixing plate on which an ePTFE specimen is positioned, thereby accelerating a bioactive metal cation by way of a potential difference formed between the bioactive metal target and the ePTFE specimen as a step of implanting a bioactive metal into an ePTFE surface without an interface by performing plasma etching using a bioactive metal target under a predetermined reaction condition.

A second aspect of the present invention provides an artificial vascular graft with a metallized surface, comprising an ePTFE substrate; and a bioactive metal implanted into a surface of the ePTFE substrate without an interface.

Hereinafter, the present invention will be described in more detail.

The present invention is designed to improve the occurrence of stenosis at a high rate due to the low blood flow rate when the ePTFE tube currently used as an artificial vascular graft material is used as an artificial vascular graft having a small diameter of 6 mm or less. When the inner diameter of the tube is treated with a plasma in order to solve this, the surface stability is low because of remaining highly reactive radicals, and there is a hassle accompanying aging treatment to improve the low surface stability. In the case of coating the tube with natural polymers or drugs such as anticoagulants, the coating is peeled off by the blood flow or the drug is quickly released, which makes it difficult to continuously obtain the desired effect, and there is a possibility that a secondary disease may be caused by the drug being released. However, the artificial vascular graft with a metallized surface, which is manufactured by implanting bioactive metal ions into the ePTFE surface by way of selective plasma etching according to the present invention not only significantly improves the adhesion and/or proliferation of vascular endothelial cells and inhibits the adhesion and/or proliferation of activated platelets, but also is not easily peeled off or worn but exhibits structural stability and can be thus stably maintained for a long period of time even in a state of being transplanted in the body since metal ions are implanted into the polymer surface and a certain depth from the surface without an interface to form a metal oxide unlike substrates coated by simple deposition of metals.

Provided is a method of manufacturing an artificial vascular graft with a metallized surface comprising an expanded polytetrafluoroethylene (ePTFE) specimen and a bioactive metal implanted into a surface of the ePTFE specimen without an interface, which comprises applying a negative voltage to a bioactive metal target in the presence of an inert gas and at the same time applying a negative voltage for bias to a fixing plate on which an ePTFE specimen is positioned and accelerating a bioactive metal cation by way of a potential difference formed between the bioactive metal target and the ePTFE specimen as a step of implanting a bioactive metal into an ePTFE surface without an interface by performing plasma etching using a bioactive metal target under a predetermined reaction condition.

The bioactive metal used in the manufacturing method of the present invention may be a material of which the ions formed during plasma generation etch the surface of the polymer substrate having a relatively low specific gravity to cause a shape change of the surface, and at the same time, the atoms formed together are deposited on the surface and remain to change the chemical composition of the surface. Non-limiting examples of the bioactive metal include tantalum, niobium, tungsten, rhenium, osmium, iridium, hafnium, platinum, or gold. Preferably, the bioactive metal may be tantalum, but is not limited thereto. Tantalum is a material exhibiting significantly high anti-corrosive property and wear resistance, and is a biocompatible material that is variously used as a corrosion resistant material for batteries and electronic devices and an implant and the like for insertion into the body or for coating these by itself or in the form of an alloy.

In the manufacturing method according to the present invention, the voltage applied to the bioactive metal target may be a negative voltage equal to or more than a threshold voltage at which metal cations starts to be emitted from the bioactive metal target. For example, when using tantalum as a target, a negative voltage in a range of 10 V to 500 V may be applied, but the voltage is not limited thereto and may be selected in consideration of the kind of bioactive metal used and the possibility of ion generation.

Furthermore, the negative voltage for bias applied to the fixing plate on which an ePTFE specimen is positioned may be a negative voltage higher than the negative voltage applied to the bioactive metal target, and for example, a negative voltage in a range of 500 V to 2000 V may be applied.

By applying a negative voltage for bias, namely, a negative voltage higher than the voltage applied to the bioactive metal, to the fixing plate on which an ePTFE specimen is positioned, it is possible to accelerate the bioactive metal cations generated from the bioactive metal target toward the ePTFE specimen and to induce implantation of metal ions into the surface of the ePTFE specimen.

In the manufacturing method of the present invention, in order to maximize the content of the bioactive metal implanted into the surface of the ePTFE specimen, the distance between the bioactive metal target and the fixing plate on which an ePTFE specimen is positioned may be adjusted, and these target and specimen may be spaced apart from each other at an interval of 5 cm to 15 cm, but the distance is not limited thereto.

For example, the manufacturing method of the present invention may be performed using a DC magnetron sputtering system equipped with a direct current power supply unit that is connected to each of the bioactive metal target and the fixing plate on which an ePTFE specimen is positioned, but is not limited thereto.

Preferably, the plasma etching may be performed using a sputter-based apparatus equipped with a vacuum chamber including a bioactive metal target and an ePTFE specimen positioned therein to be spaced apart from each other; and a direct current power supply unit connected to each of the bioactive metal target and the ePTFE specimen. Preferably, the bioactive metal ions and atoms generated by the voltage gradient may be accelerated toward the ePTFE specimen by applying a negative voltage to each of the bioactive metal target and the ePTFE specimen spaced apart to face each other, but applying a negative voltage higher than that applied to the bioactive metal target to the ePTFE specimen.

An inert gas may be injected into the vacuum chamber to provide a predetermined pressure required for the plasma process and maintain the predetermined pressure. Argon gas may be used as the inert gas, but the inert gas is not limited thereto. The pressure may be 0.5×10⁻² Torr to 5×10⁻² Torr, but is not limited thereto, and may be appropriately selected by those skilled in the art.

By selective plasma etching under the above conditions, bioactive metal ions may be implanted into the ePTFE surface and a certain depth, for example, a depth of 1 nm to 100 nm from the surface without an interface with the polymer.

The ePTFE specimen used in the manufacturing method of the present invention may be a flat specimen of which the thickness is the same as the thickness of the artificial vascular graft to be finally provided and the width and length are each equal to or more than the inner diameter and length of the artificial vascular graft to be provided.

Accordingly, the manufacturing method of the present invention may further comprise a step of bending the ePTFE specimen into a blood vessel shape so that the surface into which the bioactive metal is implanted faces inward and suturing or bonding the ePTFE specimen after the step of implanting a bioactive metal. Specifically, the ePTFE specimen, into which the bioactive metal is implanted, is preferably flat for the feature of the manufacturing method of the present invention that the ePTFE surface to be modified and the bioactive metal target to be implanted are required to be spaced apart from each other at a predetermined interval. For example, an ePTFE tube having the desired size and/or length is cut in the cylindrical direction and unfolded, a bioactive metal is implanted into the inner surface of the unfolded ePTFE, and then the unfolded ePTFE is sutured or bonded again into the form of a tube to restore the tube form, or a bioactive metal is implanted into a flat ePTFE substrate, then the flat ePTFE substrate is bent in the form of a blood vessel so that the surface into which the bioactive metal is implanted faces inward, and the bent ePTFE substrate is sutured or bonded, whereby the artificial vascular graft may be manufactured, but the artificial vascular graft is not limited thereto. At this time, the thickness of the artificial vascular graft to be manufactured may be 0.01 mm to 0.2 mm, but is not limited thereto. More specifically, the thickness and/or diameter of the artificial vascular graft may be adjusted in consideration of the blood flow rate, blood pressure, and blood vessel thickness of the vascular defect to which the artificial vascular graft is transplanted.

The process of bending the flat ePTFE substrate, into which the bioactive metal is implanted, into a blood vessel shape may be performed using a medical device for blood vessels such as a plastic cannula, but is not limited thereto. At this time, the size of the plastic cannula may be selected in consideration of the diameter of the artificial vascular graft to be manufactured.

In order to provide the artificial vascular graft in the form of a tube of which the side is closed so as to prevent blood from leaking, two edges of the substrate bent in the blood vessel shape may be sutured or bonded to each other. The suturing may be performed using any method and/or means capable of suturing the artificial vascular graft, such as suturing using a suture. The suture used for suturing ligates blood vessels to bring about a hemostatic effect or serves to support the tissue until the damaged tissue is healed, and is thus required to be inert so as not to react with the tissue, to be flexible, not to damage the tissue, and not to act as a foreign matter. As such a suture, one or more selected from the group consisting of silk, nylon, Vicryl, Prolene, and Dacron may be used. Since the silk does not slip well, the knot is hardly untied when the silk is tied only 3 to 4 times during suturing, and the silk is inexpensive, and is used for skin suturing and intestinal suturing, extensive fascial suturing and the like. The component of nylon is a polyamide polymer, and there are two types of nylon: monofilament and multifilament. Mostly monofilament nylon is used, and the thickness is thinner as the first number is larger. For the suturing, 6-0 to 10-0 monofilament nylon may be used, but the suture is not limited thereto. Meanwhile, the bonding may be performed using any method and/or means capable of bonding the artificial vascular graft using an adhesive for living tissue. As the adhesive for living tissue, materials used for the purpose of fixing tissues, suturing wounds, hemostasis, and preventing air leakage in various medical fields such as microsurgery, vascular surgery, lung surgery, plastic surgery, orthopedic surgery, and dentistry may be used without limitation. The adhesive is used *in vivo,* and is thus required to be biodegradable and not to be toxic or risky. The adhesive is intended to be inserted into the body as an artificial vascular graft, it is difficult to obtain sufficient adhesive strength since the adhesive is exposed to wet conditions by body fluids or blood after transplantation, the adhesive is required to exhibits properties such as fast adhesion and biocompatibility, and thus only limited materials may be used. As such an adhesive for living tissue, one or more selected from the group consisting of fibrin glue, gelatin glue, a polyurethane-based adhesive, and a cyanoacrylate-based adhesive may be used.

The present invention provides an artificial vascular graft with a metallized surface, which comprises an ePTFE substrate; and a bioactive metal implanted into a surface of the ePTFE substrate without an interface.

The artificial vascular graft having a metallized surface of the present invention may be manufactured by way of selective plasma etching, but is not limited thereto.

Specifically, the artificial vascular graft of the present invention may be manufactured by way of the above-described manufacturing method, but is not limited thereto.

The artificial vascular graft manufactured in this way may be one in which the bioactive metal is implanted to a depth of 1 nm to 100 nm from the ePTFE surface. It may be difficult to achieve a desired effect of improving blood compatibility when the thickness of the bioactive metal implanted layer is as thin as less than 1 nm, while it may be difficult to use the artificial vascular graft as a blood vessel required to exhibit flexibility because of the increased strength when the bioactive metal implanted layer is formed to a depth of more than 100 nm.

As described above, in the artificial vascular graft of the present invention, bioactive metal ions are implanted into the ePTFE surface without an interface. In this case, the content of the bioactive metal contained within 10 nm from the ePTFE surface is 1 at% to 50 at% on average and the content of the bioactive metal has a pattern to decrease with the depth from the ePTFE surface. As described above, since the artificial vascular graft of the present invention contains metal ions in the ePTFE substrate without an interface with a content gradient having a pattern in which the metal content decreases as the depth increases from the outermost surface, the metal layer is not peeled off but maintains a constant metal content when the artificial vascular graft is exposed to external stimulation or wet conditions and blood flow by the insertion *in vivo* as well, and thus the artificial vascular graft is structurally stable and can maintain improved properties even after a long period of time.

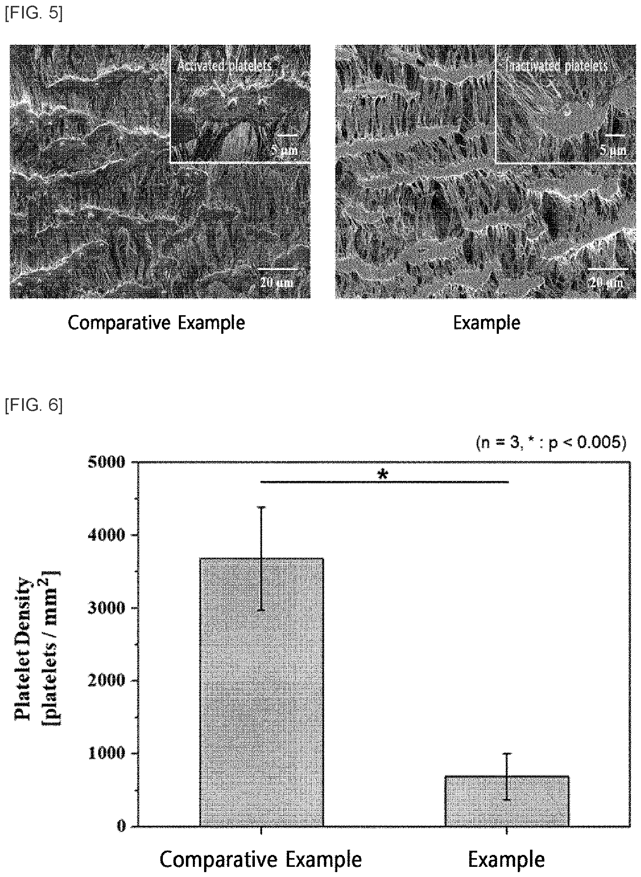

For example, the artificial vascular graft of the present invention exhibits improved blood compatibility as compared to an ePTFE substrate into which a bioactive metal is not implanted. Specifically, in the artificial vascular graft of the present invention, adhesion, proliferation, or both of adhesion and proliferation of vascular endothelial cells may be increased and adhesion, proliferation, or both of adhesion and proliferation of activated platelets may be decreased as compared to an ePTFE substrate into which a bioactive metal is not implanted. In a specific embodiment of the present invention, it has been confirmed that cell adhesion and proliferation rate significantly increase when vascular endothelial cells are cultured on the ePTFE surface into which tantalum ions are implanted by way of selective plasma etching of the present invention as compared to untreated ePTFE. When platelets are dispensed and cultured, within the same time, activated platelets widely adhered through filopodia have been found to adhere and proliferate at a high density on the untreated ePTFE surface, but a remarkably small number of platelets adhered has been observed on the ePTFE surface into which tantalum ions are implanted by way of selective plasma etching of the present invention, and these platelets have been identified as inactive platelets maintaining a disk shape (FIGS. 5 and 6).

As described above, the artificial vascular graft of the present invention may be transplanted to the defective blood vessel site *in vivo* by being sutured or bonded. For example, the artificial vascular graft of the present invention may be sutured with a vascular defect using a suture by a non-limiting suturing method known in the art, or bonded to the vascular defect using an adhesive for living tissue by a non-limiting bonding method known in the art. In this case, the types of sutures and/or adhesives for living tissue that can be used are as described above.

The artificial vascular graft according to the present invention may further comprise an antithrombotic component. As the antithrombotic component, commercially available ones may be used without limitation. For example, the antithrombotic component may be loaded in the pores of ePTFE by immersing ePTFE in a solution containing an antithrombotic component such as 4-hexylresorcinol, but is not limited thereto.

### [Detailed Description of the Invention]

Hereinafter, the present invention will be described in more detail with reference to Examples. These Examples are for explaining the present invention in more detail, and the scope of the present invention is not limited by these Examples.

### Comparative Example 1: Preparation of untreated ePTFE

Medical ePTFE artificial vascular grafts (GORE-TEX, Stretchable Vascular Grafts) commercially available from W. L. Gore & Associates, Inc. were purchased and processed to a size of 10 mm × 10 mm to facilitate subsequent experiments.

### Example 1: Preparation of ePTFE having surface treated by way of selective plasma etching

In order to implant ions of tantalum as a bioactive metal into the surface of the ePTFE specimen prepared in the size of 10 mm × 10 mm according to Comparative Example 1 by way of selective plasma etching, the ePTFE specimen and the tantalum target were positioned in the vacuum chamber of a DC magnetron sputter to be spaced about 15 cm apart from each other, and then argon gas as a sputtering gas was introduced to form a plasma in the chamber, and the degree of vacuum was formed to a level of about 10⁻² Torr. Thereafter, a negative voltage was applied to the tantalum target (purity of 99.99%) to form a plasma inside the vacuum chamber, and at the same time, a higher negative voltage was applied to the fixing plate on which the ePTFE specimen was placed to form a potential difference between the target and the fixing plate. The tantalum ions accelerated to the fixing plate by electrical attraction due to the potential difference collided with and were implanted into the surface of the ePTFE specimen, and ePTFE having a metallized surface into which tantalum ions were implanted was finally obtained. At this time, the voltage applied to the tantalum target was 500 V or less, and the treatment was completed within 1 minute in total.

### Experimental Example 1: Confirmation of change in surface structure of tantalum ion-implanted ePTFE having surface treated by way of selective plasma etching

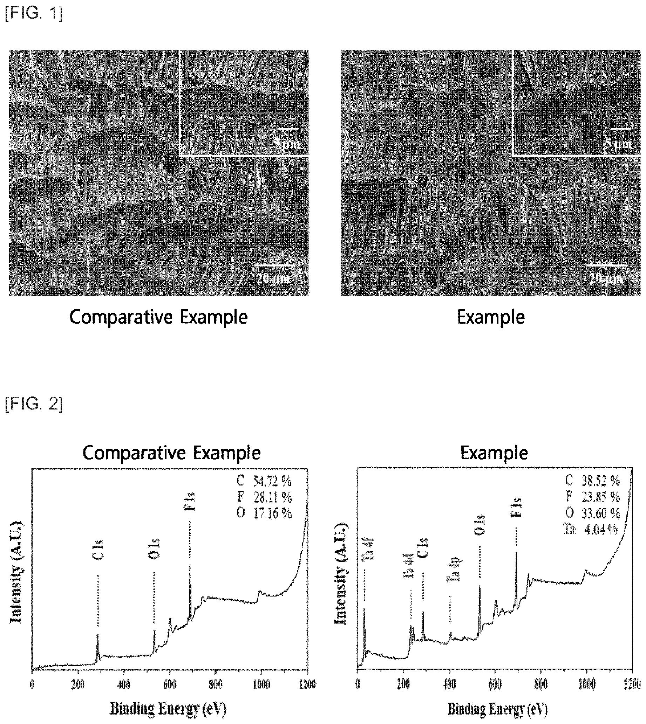

In order to confirm the changes in surface structure of ePTFE before (Comparative Example) and after (Example) subjected to the surface treatment by way of selective plasma etching according to the present invention, the surface structure of ePTFE was observed under a field emission-scanning electron microscope (FE-SEM), and the results are illustrated in FIG. 1. As illustrated in FIG. 1, it has been confirmed from the high-magnification images of the Comparative Example and Example that the porous pore structure of untreated ePTFE is stably maintained without any change in the surface structure due to the surface treatment by way of selective plasma etching.

### Experimental Example 2: Identification of chemical structure on tantalum ion-implanted ePTFE surface treated by way of selective plasma etching

In order to identify the changes in chemical species on the surface due to the implantation of tantalum ions by way of selective plasma etching confirmed in Experimental Example 1, the chemical bonding state and composition of the surface atoms of the specimens prepared according to Comparative Example 1 and Example 1 were confirmed by X-ray photoelectron spectroscopy (XPS), and the results are illustrated in FIG. 2. As illustrated in FIG. 2, any atom except carbon, oxygen, and fluorine was not detected in the Comparative Example, which was an untreated ePTFE specimen, but a tantalum atom was additionally detected (4.04%) in the Example, which was an ePTFE specimen into which tantalum ions were implanted by way of selective plasma etching.

### Experimental Example 3: Confirmation of hydrophilicity of tantalum ion-implanted ePTFE surface treated by way of selective plasma etching

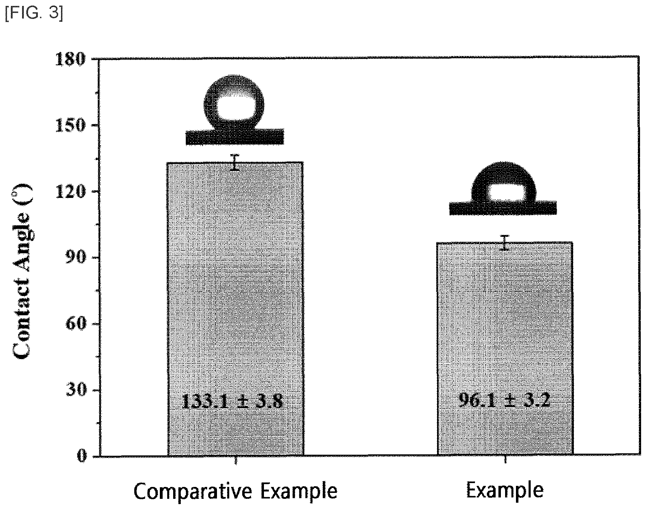

In order to confirm the changes in properties of the ePTFE surface according to the surface treatment by way of selective plasma etching, hydrophilicity, a representative surface property of polymer substrates, was confirmed. Specifically, the water contact angle on the tantalum-implanted surface of the samples of Comparative Example 1 and Example 1 was measured, and the results are illustrated in FIG. 3. As illustrated in FIG. 3, the Comparative Example, which was an untreated ePTFE specimen, was confirmed to be highly hydrophobic, having a significantly high water contact angle of about 133°, but the Example, which was an ePTFE specimen into which tantalum ions were implanted by way of selective plasma etching, had a significantly decreased water contact angle of about 96°. This indicated that hydrophilicity had been imparted to the hydrophobic ePTFE surface through the surface treatment by way of selective plasma etching.

### Experimental Example 4: Evaluation of adhesion and proliferation of vascular endothelial cell on tantalum ion-implanted ePTFE surface treated by way of selective plasma etching

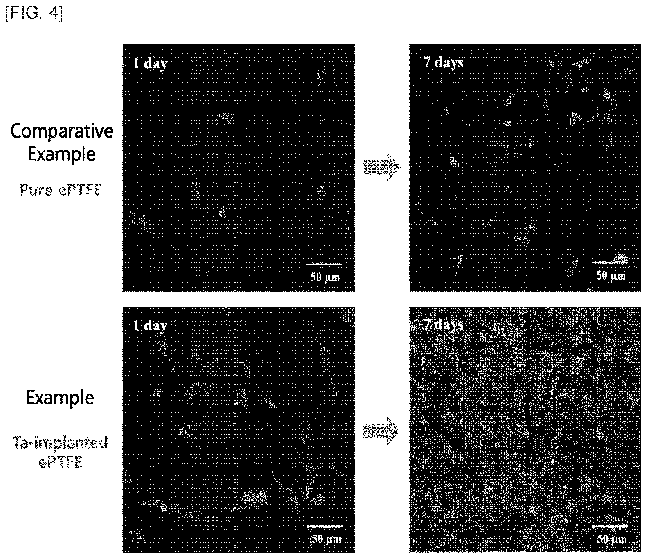

In order to confirm the effect of surface treatment by way of selective plasma etching on the blood compatibility of the ePTFE surface, vascular endothelial cells were dispensed on the specimens of Comparative Example 1 and Example 1 and cultured by way of a known method, and the degrees of adhesion and proliferation of the cells on the substrates were confirmed using a confocal microscope. Specifically, while vascular endothelial cells (ATCC, CRL-1730) were dispensed and cultured on the untreated surface and the surface treated by way of selective plasma etching of the Comparative Example and Example, the morphology of cells adhered to the surface was observed on day 1 and day 7, respectively, and the results are illustrated in FIG. 4. As illustrated in FIG. 4, the degree of proliferation was slight even when vascular endothelial cells were cultured up to the 7th day on the untreated ePTFE specimen (Comparative Example), and this is considered to be due to high hydrophobicity of the untreated ePTFE surface. On the other hand, a remarkably increased proliferation of vascular endothelial cells was observed on the ePTFE surface treated by way of selective plasma etching after 7 days of culture, and these cells spread evenly over the entire surface and adhered. This may be due to the hydrophilicity of the surface imparted through the surface treatment by way of selective plasma etching.

### Experimental Example 5: Evaluation of adhesion tendency and density of platelet on tantalum ion-implanted ePTFE surface treated by way of selective plasma etching

In order to confirm the effect of surface treatment by way of selective plasma etching on the blood compatibility of the ePTFE surface, the adhesion tendency and density of platelets on the specimens of Comparative Example 1 and Example 1 was confirmed by FE-SEM, and the results are illustrated in FIGS. 5 and 6. Specifically, platelets extracted from healthy human blood were dispensed on the untreated surface and the surface treated by way of selective plasma etching, and cultured for 1 hour, and platelets adhered to the surfaces were observed. As illustrated in FIG. 5, platelets evenly adhered to the entire region were observed on the untreated ePTFE specimen (Comparative Example), the adhered form of these platelets was observed using a high-magnification image, and it was confirmed that these platelets were activated platelets strongly adhered to the surface of the substrate. On the other hand, the number of adhered platelets observed on the ePTFE surface treated by way of selective plasma etching was significantly small, and these adhered platelets were observed using a high-magnification image and confirmed to be inactivated platelets. As illustrated in FIG. 6, the density of adhered platelets was quantitatively calculated from these images, and as a result, the density of adhered platelets in the substrate having a surface treated by way of selective plasma etching of the present invention decreased to approximately 1/6 the density of adhered platelets in the untreated ePTFE. This indicates that ePTFE having a surface treated by way of selective plasma etching according to the present invention can significantly decrease thrombus formation and/or blood coagulation as compared to untreated ePTFE.

## Claims

1. A method of manufacturing an artificial vascular graft with a metallized surface comprising an expanded polytetrafluoroethylene (ePTFE) specimen and a bioactive metal implanted into a surface of the expanded polytetrafluoroethylene specimen without an interface, the method comprising applying a negative voltage to a bioactive metal target in the presence of an inert gas and at the same time applying a negative voltage for bias to a fixing plate on which an expanded polytetrafluoroethylene specimen is positioned, thereby accelerating a bioactive metal cation by way of a potential difference formed between the bioactive metal target and the expanded polytetrafluoroethylene specimen as a step of implanting a bioactive metal into an expanded polytetrafluoroethylene surface without an interface by performing plasma etching using a bioactive metal target under a predetermined reaction condition.

2. The manufacturing method according to claim 1, wherein the bioactive metal is tantalum, niobium, tungsten, rhenium, osmium, iridium, hafnium, platinum or gold.

3. The manufacturing method according to claim 1, wherein the voltage applied to the bioactive metal target is a negative voltage equal to or more than a threshold voltage for starting to emit a metal cation from the bioactive metal target.

4. The manufacturing method according to claim 3, wherein the voltage applied to the bioactive metal target is a negative voltage in a range of 10 V to 500 V.

5. The manufacturing method according to claim 1, wherein the voltage applied to the fixing plate on which an expanded polytetrafluoroethylene specimen is positioned is a negative voltage higher than the negative voltage applied to the bioactive metal target.

6. The manufacturing method according to claim 5, wherein the voltage applied to the fixing plate on which an expanded polytetrafluoroethylene specimen is positioned is a negative voltage in a range of 500 V to 2000 V.

7. The manufacturing method according to claim 1, wherein the bioactive metal target and the fixing plate on which an expanded polytetrafluoroethylene specimen is positioned are spaced apart from each other at an interval of 5 cm to 15 cm.

8. The manufacturing method according to claim 1, which is performed using a magnetron sputtering system equipped with a direct current power supply unit that is connected to each of the bioactive metal target and the fixing plate on which an expanded polytetrafluoroethylene specimen is positioned.

9. An artificial vascular graft with a metallized surface, comprising an expanded polytetrafluoroethylene substrate; and a bioactive metal implanted into a surface of the expanded polytetrafluoroethylene substrate without an interface.

10. The artificial vascular graft according to claim 9, which is manufactured by way of selective plasma etching.

11. The artificial vascular graft according to claim 9, which is manufactured by way of the manufacturing method according to any one of claims 1 to 8.

12. The artificial vascular graft according to claim 9, wherein the bioactive metal is implanted to a depth of 1 nm to 100 nm from an expanded polytetrafluoroethylene surface.

13. The artificial vascular graft according to claim 9, wherein a content of the bioactive metal contained within 10 nm from an expanded polytetrafluoroethylene surface is 1 at% to 50 at% on average and the content of the bioactive metal has a pattern to decrease with a depth from the expanded polytetrafluoroethylene surface.

14. The artificial vascular graft according to claim 9, wherein blood compatibility is improved as compared to an expanded polytetrafluoroethylene substrate into which a bioactive metal is not implanted.

15. The artificial vascular graft according to claim 14, wherein adhesion, proliferation, or both of adhesion and proliferation of vascular endothelial cells is increased and adhesion, proliferation, or both of adhesion and proliferation of activated platelets is decreased as compared to an expanded polytetrafluoroethylene substrate into which a bioactive metal is not implanted.
